# FASCICULE DE BREVET EUROPEEN

(11) **EP 2 709 487 B1**
(45) Date de publication et mention de la délivrance du brevet: **27.04.2016**
(21) Numéro de dépôt: 12726594.0
(22) Date de dépôt: 10.05.2012
(51) Int. Cl.: A45D 40/00

(54) **COMPOSITE IMPRIME POUR DIFFUSION DE SUBSTANCES ODORANTES ET PROCEDE ET DISPOSITIF DE FABRICATION**
BEDRUCKTER VERBUNDSTOFF ZUR DIFFUSION VON DUFTSTOFFEN SOWIE HERSTELLUNGSVERFAHREN UND VORRICHTUNG
PRINTED COMPOSITE FOR DIFFUSING FRAGRANCES AND MANUFACTURING METHOD AND DEVICE

(30) Priorité: 20.05.2011 FR 1154429
(43) Date de publication de la demande: 26.03.2014
(73) Titulaire: PO-Dreux, 28101 Dreux Cedex (FR)
(72) Inventeur: LOPANDIA, Carole, F-27320 La Madeleine-de-Nonancourt (FR); FRASSAINT, Benoît, F-76130 Mont Saint Aignan (FR)
(74) Mandataire: Debay, Yves
(86) Numéro de dépôt international: PCT/EP2012/058681
(87) Numéro de publication internationale: WO 2012/159895

(56) Documents cités:
- WO-A2-2004/075933
- DE-A1- 1 532 896
- DE-A1-102004 010 015
- DE-U1- 8 405 960
- US-A- 5 439 172

## Description

La présente invention se rapporte au domaine des diffuseurs de substances odorantes et plus particulièrement au domaine des diffuseurs permettant une diffusion contrôlée et prolongée des substances odorantes qu'ils comportent.

Il devient de plus en plus courant de proposer des échantillons pour un parfum sous la forme d'un buvard imprégné et attaché dans un encart publicitaire d'un magazine. Par ailleurs, il est également courant de proposer ces échantillons sous la forme de buvard, tel qu'une carte à parfum ou une carte imprégnée, directement auprès de clients potentiels dans des parfumeries. De même, pour se démarquer de la concurrence, certaines enseignes utilisent une signature olfactive sur leurs cartes de visite ou sur courriers ou objets publicitaires destinés au lancement de nouvelles campagnes promotionnelles.

Un inconvénient majeur de ces buvards imprégnés est que leur capacité à restituer la substance odorante conduit à des diffusions non souhaitées et non contrôlées lorsqu'ils entrent en contact avec un élément réalisé en matière absorbante. C'est ainsi que ces buvards, lorsqu'ils sont destinés à être disposés entre des pages de magazines, perdent une partie de leur substance odorante en raison d'un phénomène de migration dans les pages entre lesquelles ils sont positionnés. Au-delà du premier problème de la perte d'une certaine quantité de la substance déposée, s'ajoutent d'une part une altération de la substance odorante par les encres des pages des magazines qui peuvent réagir avec la composition stockée par le buvard et d'autre part une salissure des pages des magazines entre lesquelles les buvards sont disposés.

Le brevet US 5,093,182 propose un exemple de cette application d'un parfum avec un support publicitaire imprimé. Cette application particulière est opérée de sorte que le parfum déposé sur le support publicitaire n'interagit pas avec l'encre de la surface imprimée. Pour ce faire, l'huile du parfum est préalablement mélangée en absence de phase aqueuse avec un polymère et notamment de l'éthylcellulose qui est un agent filmogène connu pour permettre un contrôle de la viscosité. Le mélange parfum/polymère est alors adapté pour être déposé sur un support imprimé par l'intermédiaire d'un dispositif offset connu. Ce parfum une fois déposé a la caractéristique de pouvoir sécher rapidement sans nécessiter de chauffage. Toutefois, une telle solution n'est pas satisfaisante dans la mesure ou la migration et/ou la diffusion du parfum déposé ne sont pas maîtrisés à l'intérieur du support imprimé ou entre plusieurs supports imprimés.

La demande de brevet JP 9076377 propose également un procédé de fixation d'un parfum sur un échantillon. Selon ce procédé, un substrat reçoit une fragrance odorante puis ce substrat odorant est recouvert d'une membrane qui vient se sceller au substrat. Cette membrane permet d'empêcher une évaporation ou diffusion précoce de la fragrance déposée lorsque le substrat est stocké. La libération des substances odorantes du substrat est alors obtenue par un utilisateur lorsque celui-ci retire la membrane de la surface du substrat. Cependant, un tel procédé ne propose pas une solution qui permette de déposer directement une substance odorante sur un substrat imprimé. De plus, si le stockage du produit permet une conservation de la substance odorante, la libération de cette substance demeure tributaire du retrait de la membrane.

Le brevet JP 2003063174 propose une solution permettant à un support de contenir deux parfums grâce à une paire de feuilles reliées le long d'une pliure. Chacune de ces feuilles comporte le long de son bord extérieur une bande recouverte d'un parfum respectif et qui est repliée et collée contre une face de la feuille en formant une poche ou une enveloppe à l'intérieur de laquelle est positionné le parfum, de façon à éviter un mélange des parfums entre eux lorsque les feuilles qui comportent chacune un parfum différent sont repliées l'une contre l'autre. Cette technique pourrait être utilisée pour limiter les problèmes d'absorptions inconsidérés par des pages de magazines. Mais en empêchant cette absorption, cette technique limite également la diffusion puisqu'elle impose à un utilisateur d'ouvrir et de déchirer la poche ou l'enveloppe pour libérer la substance odorante contenue. De plus, une fois la poche ouverte, celle-ci ne peut pas se refermer et les pages du magazine peuvent être souillées.

La demande de brevet US 2002/0013108 décrit un buvard présentant une première couche en papier ou en matériau plastique ayant une face extérieure imprimée et une deuxième couche très poreuse fixée à la face intérieure de la première couche par collage ou pression et imprégnée de parfum de façon à diffuser un parfum par frottement contre la peau. Cette solution n'est pas satisfaisante, car le parfum peut librement migrer entre les différentes couches, créant ainsi divers désordres techniques et il n'est pas prévu d'impression sur la couche buvard.

Le brevet US 6 125 614 présente une page stratifiée comprenant une feuille de papier support pliée et scellée de façon à contenir un matériau échantillon situé dans ce pli. Plus précisément, une couche barrière présentant un matériau imperméable au matériau échantillon, par exemple en polymère PET, est fixée à la feuille de papier support par un adhésif et pliée avec elle, ce qui lui permet de présenter une première partie barrière inférieure et une deuxième partie barrière supérieure entre lesquelles le matériau échantillon est disposé. Cette page stratifiée ne permet pas la libération facile du matériau échantillon et nécessite de décoller le pli.

La demande de bevet DE 10 2004 010 015 A1 décrit aussi un composite imprimé pour diffusion d'une substance odorante.

La présente invention se propose de palier un ou plusieurs inconvénients de l'art antérieur et notamment de proposer une solution qui permette de maîtriser la diffusion d'une substance odorante en contrôlant sa migration tout en empêchant une altération de ses caractéristiques odorantes par les matériaux supports employés ou par les procédés d'impression pratiqués.

La présente invention se propose également de répondre aux problématiques de production industrielle et de maîtrise de la qualité tout au long de la chaîne de production, de stockage et de distribution du produit.

Cet objectif est atteint grâce à un composite imprimé pour diffusion d'une substance odorante tel que revendiqué dans la revendication 1.

Selon une particularité de l'invention, le composite imprimé est caractérisé en ce que la couche absorbante de support-réservoir imprimable et/ou imprimée est en fibres de cellulose.

Selon une autre particularité de l'invention, le composite imprimé est caractérisé en ce qu'une première face de la couche absorbante de support-réservoir imprimable et/ou imprimée destinée à recevoir la substance odorante est macroporeuse, de type non couchée.

Selon une autre particularité de l'invention, le composite multicouche est caractérisé en ce qu'une seconde face de la couche absorbante de support-réservoir imprimable et/ou imprimée est de préférence de type couchée.

Selon une autre particularité de l'invention, le composite imprimé est caractérisé en ce que la couche barrière interne imprimée est un film polymère ou une enduction de type vernis.

Selon une autre particularité de l'invention, le composite imprimé est caractérisé en ce que la couche de barrière interne disposée contre la couche absorbante de support-réservoir imprimable et/ou imprimé forme une première barrière de migration dite interne, le composite imprimé comprenant également une couche barrière externe disposée sur la face imprimée de la couche barrière interne formant une seconde barrière de migration dite externe.

Selon une autre particularité de l'invention, le composite imprimé est caractérisé en ce que la couche barrière externe est un film polymère ou une enduction de type vernis.

Selon une autre particularité de l'invention, le composite imprimé est caractérisé en ce que la substance odorante déposée sur la première face de la couche absorbante de support-réservoir imprimable et/ou imprimée est composée d'au moins une huile essentielle.

Un autre objet de l'invention est de proposer un procédé permettant la fabrication du composite imprimé de l'invention.

Cet objet est atteint grâce à un procédé de fabrication d'un composite imprimé pour diffusion d'une substance odorante selon l'invention, caractérisé en ce que le procédé comprend notamment :
- une étape de fixation d'une couche barrière interne, tel qu'un film polymère ou une enduction de vernis, sur la deuxième face de la couche absorbante de support-réservoir imprimable et/ou imprimé qui n'est pas destinée à recevoir la substance odorante, de façon à réaliser un complexe couche absorbante de support réservoir imprimable et/ou imprimé / couche barrière interne,
- une étape de dépôt d'une substance odorante sur la première face de la couche absorbante de support-réservoir
- une étape de pliage du composite imprimé de sorte qu'une première partie de la première face (1 A) de la couche absorbante de support réservoir (1) qui n'est pas en contact avec la couche barrière interne (2) recouvre une seconde partie de cette même face (1A).

Selon une particularité de l'invention, le procédé de fabrication est caractérisé en ce que le procédé comprend également une étape de fixation d'une couche barrière externe sur la surface imprimée de la couche barrière interne.

Selon une autre particularité de l'invention, le procédé de fabrication est caractérisé en ce que l'étape de dépôt d'une substance odorante est opérée par une répartition de la substance sur une surface définie de la couche absorbante de support-réservoir imprimable et/ou imprimée.

Un autre objet de l'invention est de proposer un système permettant la mise en oeuvre du procédé de fabrication du composite imprimé de l'invention.

Cet objet est atteint grâce à un système de fabrication d'un composite imprimé pour diffusion d'une substance odorante caractérisé en ce qu'il est adapté pour la mise en oeuvre d'un procédé de fabrication selon l'invention, le système comprenant notamment :
- un poste de fixation d'une couche barrière interne, tel qu'un film polymère ou une enduction de vernis, sur la deuxième face de la couche absorbante de support-réservoir imprimable et/ou imprimé qui n'est pas destinée à recevoir la substance odorante, de façon à réaliser un complexe couche absorbante de support réservoir imprimable et/ou imprimé / couche barrière interne,
- un poste de dépôt d'une substance odorante sur la première face de la couche absorbante de support-réservoir.

L'invention, avec ses caractéristiques et avantages, ressortira plus clairement à la lecture de la description faite en référence aux dessins annexés dans lequel :
- la figure 1 représente schématiquement une superposition du bicouche (couche absorbante de support-réservoir / couche barrière interne) permettant de recevoir une substance odorante dans la couche absorbante de support-réservoir,
- la figure 2 représente schématiquement une variante du bi-couche selon l'invention dans laquelle il est plié pour éviter la diffusion de la substance odorante,
- la figure 3 représente schématiquement une variante du composite imprimé dans laquelle une couche barrière externe est fixée sur la face imprimée de la couche barrière interne,
- la figure 4 représente schématiquement une variante du composite imprimé selon l'invention dans laquelle il est plié pour éviter la diffusion de la substance odorante,
- la figure 5 représente schématiquement les différentes étapes envisageables d'un procédé de fabrication du composite de l'invention,
- la figure 6 illustre un empilement de composites imprimés lors de leur transport et/ou de leur stockage, avant pliage
- la figure 7 représente schématiquement un dispositif permettant la mise en oeuvre du procédé de fabrication selon l'invention.

Le composite de l'invention est réalisé par un ensemble composite superposé agencé pour recevoir et stocker ou emprisonner une substance odorante (4) pendant le stockage et lors des différentes étapes de la fabrication industrielle ou lors de la distribution des produits, et pour permettre sa diffusion lorsque le dépliant est ouvert par le destinataire final (lecteur de magazine ou courrier, client boutique).

Cet ensemble composite imprimé est particulièrement adapté, à titre illustratif mais non limitatif, pour parfumer des supports imprimés utilisés dans de nombreuses industries notamment celles du packaging et de la cosmétique (fabricants d'étuis, de coffrets et de cartes d'échantillons), celles des fabricants de PLV (publicité sur le lieu de vente) et celles de l'imprimerie (encarts presse, publicité adressée ou non adressée, flyers, marketing olfactif).

En particulier, il est adapté pour réaliser des encarts publicitaires ou cartes échantillons à insérer dans les feuillets d'une revue, d'un magazine et diffuser des flagrances associées à la communication produit illustrée ou imprimée sur l'encart ou la carte échantillon.

La substance odorante (4) est volatile et peut être un parfum, une huile essentielle, seule ou combinée avec un solvant, un composé aromatique, un diluant, un polymère, voire encore une résine.

La substance odorante (4) est formulée en phase liquide ou pâteuse de type huile, crème ou gel, de viscosité adaptée telle que la substance odorante (4) peut être déposée dans ou sur la couche absorbante de support réservoir imprimé et/ou imprimable (1).

La couche absorbante s'imprègne ainsi de la substance odorante par l'absorption de la substance odorante.

Selon un mode de réalisation préféré, cette substance odorante (4) recouvre au moins une partie de la surface d'une des couches situées sur l'extérieur (1 A) de l'ensemble composite, par le dépôt d'une quantité prédéfinie. Ce dépôt peut être opéré par une enduction de tout ou partie de la surface de la couche extérieure (1 A) du composite.

Préférentiellement, cette substance odorante (4) est déposée en phase liquide.

Selon un exemple de réalisation, la substance odorante (4) peut être intégrée par humidification dans la couche absorbante de support réservoir (1) permettant ainsi le stockage et la diffusion de la substance odorante (4).

Préférentiellement, la couche absorbante de support-réservoir (1) est réalisée à partir d'un papier ou d'un carton de grammage supérieur ou égal à 200 g/m².

Ce papier ou carton est en pure fibres de cellulose et est constitué d'une première face non couchée (1 A) de type macroporeuse de sorte que la substance odorante (4) puisse être absorbée par les fibres.

La surface non couchée (1 A) du carton est particulièrement adaptée pour être imprimée.

Dans ce cas, il convient d'utiliser des encres d'impression compatibles avec la substance odorante (4) afin que celles-ci n'interagissent pas ni n'altèrent les caractéristiques olfactives de la substance odorante (4). Selon un mode de réalisation préféré mais non limitatif de l'invention, les encres d'impressions utilisées sont de type UV et sont durcies par photo-polymérisation lors d'une mise en relation par rayonnement U.V. (Ultraviolets). Le séchage des encres peut également être opéré par rayonnement I.R. (Infrarouges).

Par ailleurs, ce papier ou carton en fibres de cellulose est constitué d'une seconde face préférentiellement couchée (1 B), de type microporeuse, adaptée pour recevoir et assurer une bonne fixation de la couche barrière interne (2) et pour apporter un aspect et un état de surface tendu.

Il peut s'agir d'un carton composé de pures fibres de cellulose adapté pour ne pas interagir par oxydation au contact de la substance odorante (4).

De façon remarquable, avec ce papier ou carton, et du fait de l'enchevêtrement des fibres de cellulose, la diffusion de la substance odorante (4) peut être de l'ordre de quelques jours à plusieurs semaines à l'air libre selon la quantité déposée et selon la formulation de la substance odorante (4).

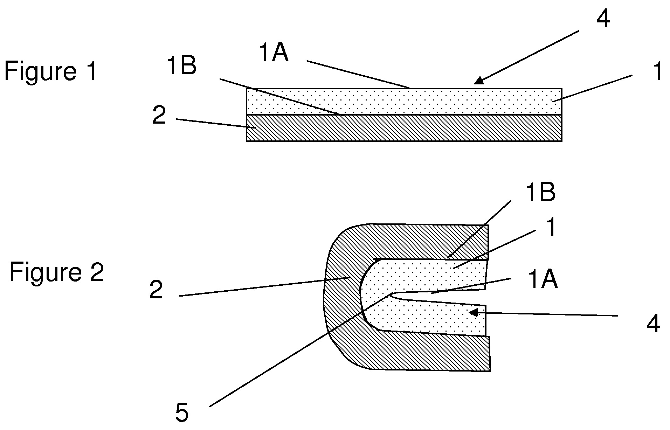

Comme illustré sur la figure 1, la couche absorbante de support-réservoir (1) est fixée par sa deuxième face (1 B) à une couche barrière interne (2), formée par exemple par un film polymère ou une enduction de vernis, qui assure une barrière de migration à la substance odorante (4) vers l'extérieur de la surface (1 B).

Selon un premier exemple de réalisation, la couche barrière interne (2) est formée par un film polymère de type PET ou OPP traité. Ainsi disposée, cette couche barrière interne (2) permet d'empêcher toute migration de la substance odorante (4) vers l'extérieur de la surface (1 B).

A titre non limitatif, lorsque la couche barrière interne (2) est un film en OPP traité, elle peut présenter une épaisseur comprise entre 12 et 20 µm et un grammage compris entre 11 et 18 g/m2. Le traitement du film polypropylène peut être opéré par un traitement au plasma ou un traitement chimique qui permettent d'obtenir une énergie de surface supérieure à 38 dynes et rendre ainsi le film OPP traité imprimable.

A titre non limitatif, lorsque la couche barrière interne (2) est un film en PET, elle peut présenter une épaisseur de 12µm et un grammage d'environ 17g/m2. L'état de surface du film PET présente une énergie de surface supérieure à 38 dynes lui permettant d'être imprimée.

Selon un second exemple de réalisation, la couche barrière interne (2) est formée par l'application d'une enduction de vernis. Ainsi déposée, cette couche barrière interne (2) permet d'empêcher toute migration de la substance odorante (4) vers l'extérieur de la deuxième surface (1 B).

A titre non limitatif, la couche barrière interne (2) est un vernis UV déposé par flexographie et présente un grammage de 4 à 6 g/m². Ce vernis est particulièrement formulé pour obtenir une énergie de surface supérieure à 38 dynes et permettre l'imprimabilité de la couche barrière interne(2).

Dans ce cas, il convient d'utiliser des encres d'impression compatibles avec la couche barrière interne fixée (2).

Selon un mode de réalisation préféré mais non limitatif de l'invention, les encres d'impressions utilisées sont de type UV et sont durcies par photo-polymérisation lors d'une mise en relation par rayonnement U.V. (Ultraviolets). Le séchage des encres peut également être opéré par rayonnement I.R. (Infrarouges).

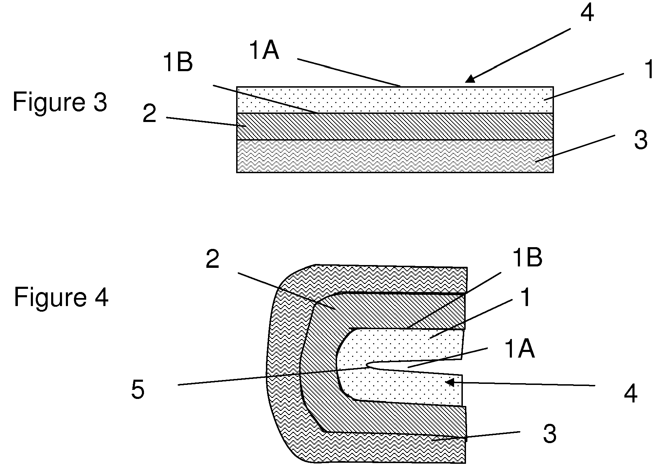

Selon un mode de réalisation de l'invention illustrée sur la figure 3, le composite imprimé pour diffusion d'une substance odorante comporte également une couche barrière externe (3) sur la surface de la couche barrière interne (2) qui n'est pas en contact avec la couche absorbante de support-réservoir (1 B).

Selon une particularité de réalisation qui n'est pas limitative de l'invention, la couche barrière externe (3) est formée par exemple par un film polymère ou une enduction de vernis.

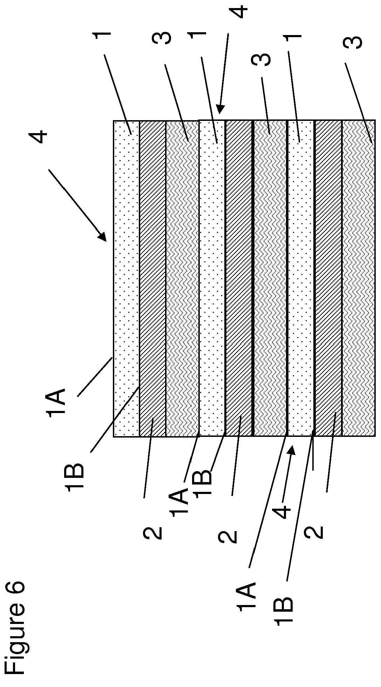

Avantageusement, cette couche barrière externe (3), permet d'empêcher la migration et la diffusion de substances odorantes (4) depuis la couche absorbante de support-réservoir imprimé et/ou imprimable (1) d'un premier composite imprimé disposé sous un second composite imprimé, en particulier lors de l'empilement, lors du stockage et lors du transport d'une pluralité de composites imprimé de l'invention, comme illustré sur la figure 6. Les deux dernières feuilles de composite imprimé situées sur le dessus de la pile peuvent être disposées en tête-bêche l'une par rapport à l'autre, c'est-à-dire en contact l'une de l'autre au niveau de leur première face extérieure (1 A), ce qui permet d'éviter ou de limiter la diffusion de la substance odorante (4) du fait de l'imperméabilité de la couche barrière externe (3) située à l'extrémité de la pile.

Cette couche barrière externe (3) peut, par exemple, selon ses caractéristiques, présenter une surface du composite imprimé de l'invention qui est en mesure d'être collée contre un support. Cette couche barrière externe (3) peut ainsi être collée et permet la fixation du composite imprimé sur un support tel que, par exemple et de façon non limitative, un magazine ou un élément publicitaire, ou encore un élément de présentation de type P.L.V. (Publicité sur Lieu de Vente).

Dans une alternative de réalisation illustrée sur la figure 4, le composite imprimé de l'invention comprend également au moins un axe d'articulation (5) pour permettre le recouvrement d'une première partie de la face extérieure (1 A) de la couche absorbante de support réservoir (1) qui n'est pas en contact avec la couche barrière interne (2) sur une seconde partie de cette même face extérieure (1 A). Toutes les couches du bicouche (couche absorbante de support réservoir (1), couche barrière interne(2)), et du tricouche (couche absorbante de support réservoir (1), couche barrière interne(2), couche barrière externe (3)) sont pliées conjointement, comme illustré sur les figures 2 et 4.

Cet axe d'articulation (5) peut être réalisé, par exemple, par un rainage ou un pliage axial. Ainsi, lorsque la couche absorbante de support réservoir imprimé et/ou imprimable (1) comprend une substance odorante (4), le recouvrement par repliement l'une sur l'autre des deux parties de la face de la couche absorbante de support réservoir (1) dans une position fermée permet de limiter voire de stopper la diffusion de la substance odorante (4).

La libération par diffusion de la substance odorante (4) est alors effectuée par un utilisateur qui ouvre et/ou déplie le composite imprimé de l'invention (ouverture du magazine, ouverture de la carte échantillon, ouverture d'un dépliant de marketing direct...).

Ainsi, le composite imprimé de l'invention permet de réaliser un support pour la diffusion d'une substance odorante (4) qui soit en mesure d'être associé avec un élément de communication visuelle imprimé (texte, marque, message publicitaire, etc.) tant sur la partie formée par la couche absorbante de support-réservoir (1) que sur la partie formée par la couche barrière interne (2).

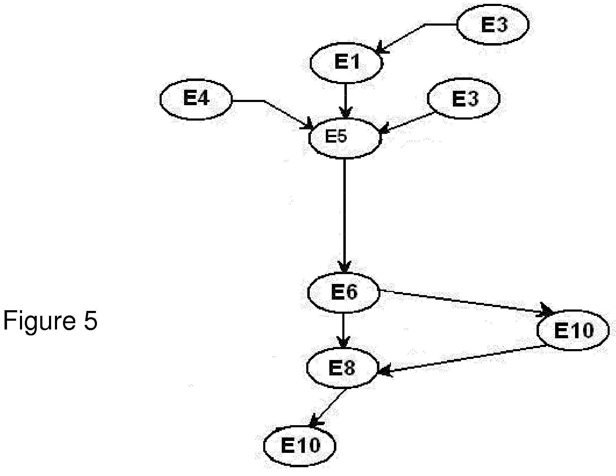

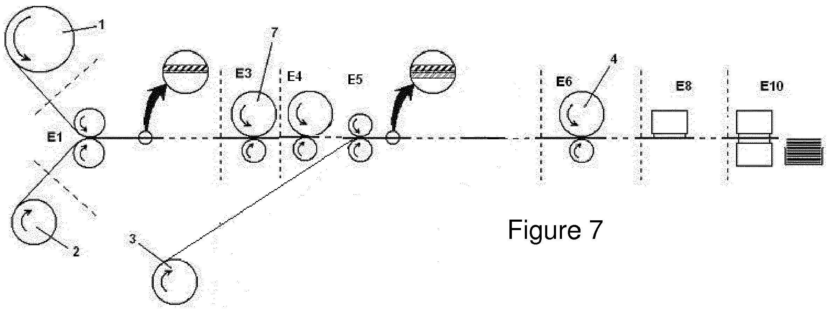

La fabrication de ce composite imprimé de l'invention impose plusieurs étapes successives dans un procédé de fabrication illustré sur les figures 5 et 7, et dont l'ordre peut être différent pour certaines d'entre elles.

Ainsi, le procédé de fabrication du composite imprimé de l'invention comprend notamment :
- une étape de fixation (E1) d'une couche barrière interne (2), tel qu'un film polymère ou une enduction de vernis, sur la deuxième face (1 B) de la couche absorbante de support-réservoir imprimable et/ou imprimé (1) qui n'est pas destinée à recevoir la substance odorante (4), de façon à réaliser un complexe couche absorbante de support réservoir imprimable et/ou imprimé (1) / couche barrière interne (2),
- une étape de dépôt (E6) d'une substance odorante (4) sur la face (1 A) de la couche absorbante de support-réservoir (1),

L'étape de fixation (E1) de la couche absorbante de support-réservoir (1) sur la couche barrière interne (2) est opérée par pelliculage et/ou complexage et/ou enduction de vernis.

L'étape de dépôt (E6) quant à elle peut être effectuée par enduction, impression de la surface de la couche absorbante de support-réservoir (1) par la substance odorante (4) destinée à être diffusée. Cette étape de dépôt peut faire intervenir des techniques d'impression par sérigraphie, flexographie ou offset. Ces techniques permettent une répartition de la substance odorante (4) sur toute ou partie de la surface de la couche absorbante de support-réservoir (1) et permettent de faire varier la quantité de substance odorante (4) déposée.

Lors de la fabrication du composite imprimé, comme illustré sur les figures 3 et 5, il peut être nécessaire d'opérer une impression (E3) sur la face (1 A) de la couche absorbante de support-réservoir (1) par un dispositif d'impression approprié (7). Cette impression (E3) peut s'effectuer soit au préalable à l'étape de fixation (E1) de la couche barrière interne (2) sur la face couche absorbante de support-réservoir (1), soit postérieurement à cette étape, mais préférentiellement au préalable de l'étape de dépôt (E6) de la substance odorante (4) sur la couche absorbante de support-réservoir (1).

De même, le procédé comprend une étape d'impression (E4) de la couche barrière interne (2) par un dispositif et/ou système d'impression approprié. Cette étape d'impression (E4) est effectuée après l'étape (E1) de fixation de la couche absorbante de support-réservoir (1) sur la couche barrière interne (2). Cette étape d'impression (E4) est effectuée au préalable à l'étape de dépôt (E6) de la substance odorante (4) sur la couche absorbante de support-réservoir (1).

De même, au préalable à l'étape de dépôt (E6) d'une substance odorante (4) sur la couche absorbante de support-réservoir (1), le procédé peut comprendre une étape de fixation (E5) d'une couche barrière externe (3) sur la couche barrière (2) imprimée.

Le composite imprimé étant réalisé, et la substance odorante (4) étant éventuellement déposée (E6), le procédé peut encore comprendre une étape de façonnage (E8). Cette étape de façonnage (E8) consiste, par exemple, en une ou plusieurs opérations d'une liste comprenant par exemple une dorure, un gaufrage, une découpe, un rainage,...

Selon un premier mode de réalisation particulier qui n'est pas limitatif du principe de l'invention, l'homogénéité de la répartition et la détermination de la surface destinée à être recouverte par la substance odorante (4) sont définies par un écran formant un tamis de maille variable (principe similaire à la technique d'impression de type sérigraphie). Par exemple, à titre non limitatif, il est possible de déposer par ce procédé une quantité de substance odorante de 12 à 20 g/m2.

Selon un second mode de réalisation particulier qui n'est pas limitatif du principe de l'invention, l'homogénéité de répartition peut également faire intervenir un anilox, qui est un cylindre comportant sur sa surface périphérique différentes formes de gravures ou d'alvéoles qui, selon leurs formes et volumes respectifs et en combinaison avec un blanchet, participent au dosage de la quantité de substance odorante (4) destinée à être déposée sur la couche absorbante de support-réservoir (1) (principe similaire à la technique d'impression de type flexographie). Par exemple, à titre non limitatif, il est possible de déposer par ce procédé une quantité de substance odorante (4) de 3 à 10 g/m2.

Selon un troisième mode de réalisation particulier, une surface métallique formant par exemple une plaque est utilisée pour transférer la substance odorante (4) sur une surface élastomère formant par exemple un blanchet. La surface élastomère est positionnée en contact avec la surface de la couche absorbante de support-réservoir (1) (principe similaire à la technique d'impression de type offset). Par exemple, à titre non limitatif, il est possible de déposer par ce procédé une quantité de substance odorante (4) de l'ordre d'un ou de quelques g/m2.

Le composite imprimé étant réalisé et la substance odorante (4) étant déposée, le système de l'invention permet un transport et un stockage du composite imprimé sous forme de feuilles ou aplats empilés de telle sorte que la diffusion ou la migration de la substance odorante (4) sont contrôlés.

Selon un mode de réalisation préférentiel, le procédé de l'invention comprend également une étape de pliage (E10) du composite imprimé de sorte qu'une première partie de la face extérieure (1 A) de la couche absorbante de support réservoir (1) qui n'est pas en contact avec la couche de barrière interne (2) recouvre une seconde partie de cette même face extérieure (1 A) de la couche absorbante de support réservoir (1). Préférentiellement, cette opération de pliage (E10) est opérée postérieurement à l'étape de dépôt (E6) d'une substance odorante (4) sur le composite imprimé. Eventuellement, l'étape de dépôt (E6) de la substance odorante (4) sur la couche absorbante de support réservoir (1) peut être réalisée après toutes les autres étapes de fabrication dans un point de vente lors de la distribution ou de la présentation finale du composite imprimé au client.

Par ailleurs, le système de fabrication, permettant la mise en oeuvre du procédé de l'invention, peut être formé par un unique dispositif ou une pluralité de dispositifs séparés entre eux. Ces dispositifs comportent alors un ou plusieurs postes arrangés pour effectuer au moins une des différentes opérations ou étapes du procédé de fabrication du composite imprimé de l'invention.

Selon un mode de réalisation particulier mais qui n'est pas limitatif de l'invention illustré sur la figure 7, le système de l'invention comporte une pluralité de postes, chacun étant adapté pour permettre la réalisation d'au moins une des étapes : fixation de couches barrières au support du composite (E1, E5), impression (E3, E4), dépôt de substance odorante (E6), ou façonnage (E8) du procédé de fabrication de l'invention.

Selon un mode de réalisation préféré qui n'est pas limitatif de l'invention, le dispositif comprend également une unité centrale qui permet la coordination du fonctionnement des différents postes du système. Cette coordination peut concerner aussi bien simplement deux postes que l'ensemble des postes du système, l'unité centrale pouvant contrôler au moins un poste du dispositif.

Comme déjà décrit précédemment, la figure 5 qui propose une représentation schématique des différentes étapes du procédé de fabrication du composite imprimé de l'invention montre clairement que certaines étapes du procédé, notamment les étapes d'impression (E3, E4), peuvent être effectuée à des moments différents du procédé par rapport aux étapes principales de formation du bi- couche (E1) et de dépôt de la substance odorante (E6) du composite.

Le système de mise en oeuvre du procédé de l'invention comporte une pluralité de postes dont chacun est organisé, arrangé ou conçu pour opérer respectivement au moins une étape du procédé de l'invention précédemment décrit. Selon un mode de réalisation non limitatif de l'invention, les postes du système de mise en oeuvre du procédé de l'invention peuvent être positionnés sur un ou plusieurs sites de production différents. Les produits obtenus au niveau de chacun de ces postes peuvent alors être transportés d'un site à l'autre pour que le procédé puisse continuer d'être mis en oeuvre. De même, le système de l'invention peut intégrer une unité centrale qui permet la gestion et la coordination du système dans la production du composite.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration mais peuvent être modifiés dans le domaine défini par la portée des revendications jointes.

## Revendications

1. Composite imprimé pour diffusion d'une substance odorante (4) comprenant :
- une couche absorbante de support-réservoir imprimable et/ou imprimée (1), adaptée pour recevoir une substance odorante (4) destinée à être diffusée, le composite comprenant en outre :
- une couche barrière interne imprimée (2), disposée contre une face (1 B) de la couche absorbante de support-réservoir imprimable et/ou imprimée (1), pour contrôler la migration et/ou la diffusion de la substance odorante (4)
- au moins un axe d'articulation (5) pour permettre le recouvrement d'une première partie de la face (1A) de la couche absorbante de support réservoir imprimable et/ou imprimé (1) qui n'est pas en contact avec la couche barrière interne (2) sur une seconde partie de cette même face (1A), caractérisé en ce qe toutes les couches sont pliées conjointement selon l'axe d'articulation en recouvrant par repliement l'une sur l'autre les deux parties de la face de la couche absorbante du support réservoir dans une position fermée.

2. Composite imprimé selon la revendication 1, **caractérisé en ce que** la couche absorbante de support-réservoir imprimable et/ou imprimée (1) est en fibres de cellulose.

3. Composite imprimé selon la revendication 2, **caractérisé en ce qu'**une première face (1A) de la couche absorbante de support-réservoir imprimable et/ou imprimée (1) destinée à recevoir la substance odorante (4) est macroporeuse, de type non couchée.

4. Composite imprimé selon la revendication 1 à 3, **caractérisé en ce qu'**une seconde face (1B) de la couche absorbante de support-réservoir imprimable et/ou imprimée (1) est de préférence de type couchée.

5. Composite imprimé selon la revendication 1, **caractérisé en ce que** la couche barrière interne imprimée (2) est un film polymère ou une enduction de type vernis.

6. Composite imprimé selon l'une des revendications précédentes, **caractérisé en ce que** la couche de barrière interne (2) disposée contre la couche absorbante de support-réservoir imprimable et/ou imprimé (1) forme une première barrière de migration dite interne, le composite imprimé comprenant également une couche barrière externe (3) disposée sur la face imprimée de la couche barrière interne (2) formant une seconde barrière de migration dite externe.

7. Composite imprimé selon la revendication 6, **caractérisé en ce que** la couche barrière externe (3) est un film polymère ou une enduction de type vernis.

8. Composite imprimé selon l'une des revendications précédentes, **caractérisé en ce que** la première face (1A) de la couche absorbante de support-réservoir imprimable et/ou imprimée (1) est apte à recevoir une substance odorante (4) composée d'au moins une huile essentielle.

9. Composite imprimé selon la revendication 1, **caractérisé en ce que** l'axe d'articulation est réalisée par rainage ou par pliage axial.

10. Procédé de fabrication d'un composite imprimé pour diffusion d'une substance odorante selon l'une des revendications 1 à 9, **caractérisé en ce que** le procédé comprend notamment :
- une étape de fixation d'une couche barrière interne (2), tel qu'un film polymère ou une enduction de vernis, sur la deuxième face (1B) de la couche absorbante de support-réservoir imprimable et/ou imprimé (1) qui n'est pas destinée à recevoir la substance odorante (4), de façon à réaliser un complexe couche absorbante de support réservoir imprimable et/ou imprimé (1) / couche barrière interne (2),
- une étape de dépôt d'une substance odorante (4) sur la première face (1A) de la couche absorbante de support-réservoir (1),
- une étape de pliage du composite imprimé de sorte qu'une première partie de la première face (1A) de la couche absorbante de support réservoir (1) qui n'est pas en contact avec la couche barrière interne (2) recouvre une seconde partie de cette même face (1A).

11. Procédé de fabrication selon la revendication 10, **caractérisé en ce que** le procédé comprend également une étape de fixation d'une couche barrière externe (3) sur la surface imprimée de la couche barrière interne (2).

12. Procédé de fabrication selon l'une des revendications 10 à 11, **caractérisé en ce que** l'étape de dépôt d'une substance odorante (4) est opérée par une répartition de la substance sur une surface définie de la couche absorbante de support-réservoir imprimable et/ou imprimée (1).

13. Système de fabrication d'un composite imprimé pour diffusion d'une substance odorante (4) **caractérisé en ce qu'**il est adapté pour la mise en oeuvre d'un procédé de fabrication selon une des revendications 10 à 12, le système comprenant notamment :
- un poste de fixation d'une couche barrière interne (2), tel qu'un film polymère ou une enduction de vernis, sur la deuxième face (1B) de la couche absorbante de support-réservoir imprimable et/ou imprimé (1) qui n'est pas destinée à recevoir la substance odorante (4), de façon à réaliser un complexe couche absorbante de support réservoir imprimable et/ou imprimé (1) / couche barrière interne (2),
- un poste de dépôt d'une substance odorante (4) sur la première face (1A) de la couche absorbante de support-réservoir (1),

## Patentansprüche

1. Verbundstoff, bedruckt zur Diffusion eines Duftstoffs (4), der Folgendes umfasst:
- eine absorbierende Schicht aus bedruckbarem und/oder bedrucktem Trägerreservoir zum Aufnehmen eines zu diffundierenden Duftstoffs (4),
wobei der Verbundstoff ferner Folgendes umfasst:
- eine bedruckte innere Schichtbarriere (2), die an einer Fläche (1 B) der absorbierenden Schicht aus bedruckbarem und/oder bedrucktem Trägerreservoir (1) angeordnet ist, zum Regulieren der Migration und/oder der Diffusion des Duftstoffs (4),
- wenigstens eine Gelenkachse (5) zum Zulassen des Bedeckens eines ersten Teils der Fläche (1A) der absorbierenden Schicht aus bedruckbarem und/oder bedrucktem Trägerreservoir (1), der nicht mit der inneren Barrierenschicht (2) auf einem zweiten Teil derselben Fläche (1A) in Kontakt kommt, **dadurch gekennzeichnet, dass** alle Schichten gemeinsam gemäß der Gelenkachse gefaltet sind und durch Aufeinanderfalten der beiden Teile der Fläche der absorbierenden Schicht des Trägerreservoirs in einer geschlossenen Position bedeckt werden.

2. Bedruckter Verbundstoff nach Anspruch 1, **dadurch gekennzeichnet, dass** die absorbierende Schicht aus bedruckbarem und/oder bedrucktem Trägerreservoir (1) aus Zellulosefasern besteht.

3. Bedruckter Verbundstoff nach Anspruch 2, **dadurch gekennzeichnet, dass** eine erste Fläche (1A) der absorbierenden Schicht aus bedruckbarem und/oder bedrucktem Trägerreservoir (1) zum Aufnehmen des Duftstoffs (4) makroporös ist, vom unbeschichteten Typ.

4. Bedruckter Verbundstoff nach den Ansprüchen 1 bis 3, **dadurch gekennzeichnet, dass** eine zweite Fläche (1B) der absorbierenden Schicht aus bedruckbarem und/oder bedrucktem Trägerreservoir (1) vorzugsweise vom beschichteten Typ ist.

5. Bedruckter Verbundstoff nach Anspruch 1, **dadurch gekennzeichnet, dass** die bedruckte interne Barrierenschicht (2) ein Polymerfilm oder ein Überzug des Firnistyps ist.

6. Bedruckter Verbundstoff nach einem der vorangegangenen Ansprüche, **dadurch gekennzeichnet, dass** die an der absorbierenden Schicht aus bedruckbarem und/oder bedrucktem Trägerreservoir (1) angeordnete innere Barrierenschicht (2) eine erste, also innere, Migrationsbarriere bildet, wobei der bedruckte Verbundstoff auch eine äußere Barrierenschicht (3) umfasst, die auf der bedruckten Fläche der inneren Barrierenschicht (2) angeordnet ist und eine zweite, also äußere, Migrationsbarriere bildet.

7. Bedruckter Verbundstoff nach Anspruch 6, **dadurch gekennzeichnet, dass** die äußere Barrierenschicht (3) eine Polymerfolie oder ein Überzug des Firnistyps ist.

8. Bedruckter Verbundstoff nach einem der vorangegangenen Ansprüche, **dadurch gekennzeichnet, dass** die erste Fläche (1A) der absorbierenden Schicht aus bedruckbarem und/oder bedrucktem Trägerreservoir (1) einen wenigstens ein essentielles Öl beinhaltenden Duftstoff (4) aufnehmen kann.

9. Bedruckter Verbundstoff nach Anspruch 1, **dadurch gekennzeichnet, dass** die Gelenkachse durch Riffelung oder durch axiale Faltung realisiert wird.

10. Verfahren zur Herstellung eines bedruckten Verbundstoffs zur Diffusion eines Duftstoffs nach einem der Ansprüche 1 bis 9, **dadurch gekennzeichnet, dass** das Verfahren insbesondere Folgendes beinhaltet:
- einen Schritt des Fixierens einer inneren Barrierenschicht (2), wie einen Polymerfilm oder einen Firnisüberzug auf der zweiten Fläche (1B) der absorbierenden Schicht aus bedruckbarem und/oder bedrucktem Trägerreservoir (1), die nicht zum Aufnehmen des Duftstoffs (4) bestimmt ist, zum Realisieren einer komplexen absorbierenden Schicht aus bedruckbarem und/oder bedrucktem Trägerreservoir (1) und innerer Barrierenschicht (2),
- einen Schritt des Absetzens eines Duftstoffs (4) auf die erste Fläche (1A) der absorbierenden Trägerreservoirschicht (1),
- einen Schritt des Faltens des bedruckten Verbundstoffs, so dass ein erster Teil der ersten Fläche (1A) der absorbierenden Trägerreservoirschicht (1), die nicht mit der inneren Barrierenschicht (2) in Kontakt kommt, einen zweiten Teil derselben Fläche (1A) bedeckt.

11. Herstellungsverfahren nach Anspruch 10, **dadurch gekennzeichnet, dass** das Verfahren auch einen Schritt des Fixierens einer äußeren Barrierenschicht (3) auf der bedruckten Fläche der inneren Barrierenschicht (2) beinhaltet.

12. Herstellungsverfahren nach einem der Ansprüche 10 und 11, **dadurch gekennzeichnet, dass** der Schritt des Absetzens eines Duftstoffs (4) durch eine Verteilung des Stoffs auf einer definierten Oberfläche der absorbierenden bedruckbaren und/oder bedruckten Trägerreservoirschicht (1) erfolgt.

13. System zur Herstellung eines bedruckten Verbundstoffs zur Diffusion eines Duftstoffs (4), **dadurch gekennzeichnet, dass** es zum Ausführen eines Herstellungsverfahrens nach einem der Ansprüche 10 bis 12 angepasst ist, wobei das System insbesondere Folgendes umfasst:
- eine Station zum Fixieren einer inneren Barrierenschicht (2), wie zum Beispiel einen Polymerfilm oder einen Firnisüberzug, auf der zweiten Fläche (1 B) der absorbierenden bedruckbaren und/oder bedruckten Trägerreservoirschicht (1), die nicht den Duftstoff (4) aufnehmen soll, um eine komplexe absorbierende bedruckbare und/oder bedruckte Trägerreservoirschicht (1) und innere Barrierenschicht (2) zu realisieren,
- eine Station zum Absetzen eines Duftstoffs (4) auf die erste Fläche (1A) der absorbierenden Trägerreservoirschicht (1).

## Claims

1. Printed composite for diffusion of a fragranced substance (4), **characterised in that** the composite comprises:
- an absorbent printable and/or printed substrate-reservoir layer (1) adapted to receive a fragranced substance (4) intended to be diffused,
said composite also comprising:
- a printed inner barrier layer (2) disposed against one face (1B) of the absorbent printable and/or printed substrate-reservoir layer (1) in order to control the migration and/or the diffusion of the fragranced substance (4);
- at least one hinge axis (5) to allow the overlapping of a first part of the face (1A) of the absorbent layer of printable and/or printed substrate-reservoir (1) which is not in contact with the inner barrier layer (2), over a second part of this same face (1A),
**characterised in that** all the layers are folded jointly along the hinge axis by overlapping by folding the two parts of the face of the absorbent layer of substrate-reservoir over one another into a closed position.

2. Printed composite according to claim 1, **characterised in that** the absorbent layer of printable and/or printed substrate-reservoir (1) is made of cellulose fibres.

3. Printed composite according to claim 2, **characterised in that** a first face (1A) of the absorbent layer of printable and/or printed substrate-reservoir (1) intended to receive the fragranced substance (4) is macroporous, of an uncoated type.

4. Printed composite according to claim 1 to 3, **characterised in that** a second face (1B) of the absorbent layer of printable and/or printed substrate-reservoir (1) is preferably of a coated type.

5. Printed composite according to claim 1, **characterised in that** the printed inner barrier layer (2) is a polymer film or a varnish-type coating.

6. Printed composite according to according to one of the preceding claims, **characterised in that** the inner barrier layer (2) disposed against the absorbent layer of printable and/or printed substrate-reservoir (1) forms a first so-called inner barrier against migration, the printed composite also comprising an outer barrier layer (3) disposed on the printed face of the inner barrier layer (2) forming a second so-called outer barrier against migration.

7. Printed composite according to claim 6, **characterised in that** the outer barrier layer (3) is a polymer film or a varnish-type coating.

8. Printed composite according to one of the preceding claims, **characterised in that** the fragranced substance (4) deposited on the first face (1A) of the absorbent layer of printable and/or printed substrate-reservoir (1) is composed of at least one essential oil.

9. Printed composite according to claim 1, **characterised in that** the hinge axis is produced by scoring or by axial folding.

10. Method of manufacture of a printed composite for diffusion of a fragranced substance according to one of claims 1 to 9, **characterised in that** the method comprises in particular:
- a step of fixing an inner barrier layer (2), such as a polymer film or a varnish coating, onto the second face (1B) of the absorbent layer of printable and/or printed substrate-reservoir (1) which is not intended to receive the fragranced substance (4), in such a way as to obtain a complex of absorbent layer of printable and/or printed substrate-reservoir (1)/inner barrier layer (2);
- a step of depositing a fragranced substance (4) on the first face (1A) of the absorbent layer of substrate-reservoir (1); and
- a step of folding the printed composite so that a first part of the first face (1A) of the absorbent layer of substrate-reservoir (1) which is not in contact with the inner barrier layer (2) overlaps a second part of this same surface (1A).

11. Method of manufacture according to claim 10, **characterised in that** the method also comprises a step of fixing an outer barrier layer (3) onto the printed surface of the inner barrier layer (2).

12. Method of manufacture according to one of claims 10 to 11, **characterised in that** the step of depositing a fragranced substance (4) is performed by distribution of the substance over a defined surface of the absorbent layer of printable and/or printed substrate-reservoir (1).

13. System of manufacture of a printed composite for diffusion of a fragranced substance (4), **characterised in that** it is adapted for implementing a method of manufacture according to one of claims 10 to 12, the system comprising in particular:
- a fixing station for fixing an inner barrier layer (2) such as a polymer film or a varnish coating onto the second face (1B) of the absorbent layer of printable and/or printed substrate-reservoir (1) which is not intended to receive the fragranced substance (4), in such a way as to obtain a complex of absorbent layer of printable and/or printed substrate-reservoir (1)/inner barrier layer (2); and
- a station for deposition of a fragranced substance (4) on the first face (1A) of the absorbent layer of substrate-reservoir (1).
